# EUROPEAN PATENT SPECIFICATION

(11) **EP 2 450 013 B1**
(45) Date of publication and mention of the grant of the patent: **12.07.2017**
(21) Application number: 11183229.1
(22) Date of filing: 23.11.2007
(51) Int. Cl.: A61F 13/20, A61F 13/42

(54) **Sanitary tampon**
Hygienetampon
Tampon périodique

(30) Priority: 24.11.2006 US 860798 P; 12.01.2007 US 880023 P; 01.02.2007 US 898710 P; 30.03.2007 US 920776 P; 02.04.2007 US 907411 P; 29.06.2007 US 929490 P
(43) Date of publication of application: 09.05.2012
(62) Divisional of application: 07866620.3
(73) Proprietor: Intigena (Schweiz) AG, 6300 Zug (CH)
(72) Inventor: Chaffringeon, Bernard, Dubai UAE (AE)
(74) Representative: Von Rohr Patentanwälte Partnerschaft mbB

(56) References cited:
- WO-A-2006/005069
- FR-A1- 2 803 208
- JP-A- 2004 097 304
- US-A- 4 185 631

## Description

The present invention relates to a sanitary tampon with the features of the introductory part of claim 1.

Many sanitary articles exist, especially for absorbing menstrual fluids, in particular catamenial and/or sanitary tampons made of absorbent material in the form of sheets and/or bands that are folded and/or rolled up, using techniques well known to persons skilled in the art, in order to form tampons that are inserted, with or without applicators, into the vaginas of the users.

However, although there are many models made of various materials and with absorption capacities that vary according to the particular requirements and losses, problems associated with leaking and/or soiling still exist that are due either to absorption capacities that are too low or to problems of volume, or even poor adherence to the vaginal wall, or a drainage phenomenon due in particular to the withdrawal devices, namely the threads and/or strings designed to remove the used tampon.

A problem also exists at the moment of withdrawal when the withdrawal devices, namely the threads and/or strings designed to remove the used tampon, and the vaginal wall can exert a pressure on the tampons and cause drying, which leads to a flow of fluid that soils the user's hands. Moreover, given that the lower part of the tampon, also called the proximal part, is greatly enlarged, this part has a tendency to cause injuries by rubbing on the vaginal wall, especially in the area of muscular narrowing of the vaginal wall.

Many devices have already been proposed. For example, patent application WO 2006/094753 discloses a tampon with an impermeable zone formed by a plastic film, and zones of variable absorption in order to avoid drying of the vaginal walls. Moreover, US 3,693,622 A discloses tampons and other sanitary articles which, at their ends, comprise zones impregnated with fluid-repelling compositions.

The devices described in these documents nevertheless have numerous disadvantages, such as the possibility of the fluid-repelling compositions migrating into the whole of the tampon and/or the sanitary article after production, the possibility of the fluid-repelling compositions being transferred to the user's finger at the moment of insertion of the tampon, and especially the impossibility of limiting the drying effect during withdrawal. Moreover, the disclosed devices do not solve the problem of the shaping and adaptation of a tampon such that it better matches the shape of the vaginal walls,

US 7,060,057 A discloses tampons comprising nonabsorbent zones arranged on the outer surface of the tampon that permit increased comfort at the interface between the tampon and the vaginal walls.

US 7,097,638 A discloses tampons comprising zones that form reinforcements on the outer walls of the tampon in order to improve comfort while at the same time maintaining the absorption capacities of the tampons.

US 3,965,905 A discloses tampons formed of a plurality of absorbent product parts connected by a withdrawal cord.

These different devices, however, do not significantly improve absorption while at the same time improving comfort and retaining other important properties. The tampon described in US 3,965,905 A, for example, does not provide a tampon that is sufficiently cohesive to permit its insertion without an applicator.

US 2003/0097108 A1 describes a tampon comprising an absorbent compressed body whose outer face is largely covered by an absorbent shroud that forms a skirt beyond the proximal end of the body of the tampon.

US 2005/0096620 A1 describes a tampon obtained by rolling up a layer of absorbent material, the layer having an edge intended to constitute the proximal end after rolling, and being covered by an absorbent shroud before rolling.

US 4,211,225 A describes a tampon permitting improved comfort during its withdrawal. For this purpose, a first shroud and then a second shroud are attached to the body of the tampon and are fixed in the area of the distal end of the tampon. The first shroud is designed such that the frictional forces generated during withdrawal between the body of the tampon and the first shroud are less than the frictional forces between the second shroud and the surrounding tissues.

The prior art forming the starting point of the invention is disclosed in US 2002/0173760 A1.

Starting from above mentioned prior art it is an object of the present invention to provide a sanitary tampon which comprises means for limiting the expansion and/or the absorption when the tampon comes into contact with a fluid that is to be absorbed.

The above mentioned object is met with a sanitary tampon comprising the features of the introductory part of claim 1 and further the features of the characterizing part of claim 1.

Preferred modifications and improvements of such sanitary tampon are the subject matter of the dependent claims.

The disclosure is also directed to tampons having a withdrawal thread that is also at least partially impregnated with a solid hydrophobic substance whose melting point is greater than 37°C.

By using a solid hydrophobic substance whose melting point is greater than 37°C, the zones in which the expansion and/or the absorption are limited are permanent, that is to say, the zones are nondeformable or are only minimally deformable, even when the tampon is saturated.

A solid hydrophobic substance whose melting point is greater than 37°C may have certain properties that are similar to those of beeswax, that is to say a substance whose plasticity permits malleability at room temperature, whose viscosity is low when the substance is melted, and that is totally hydrophobic.

In embodiments, the hydrophobic substance is chosen from the group consisting of hydrogenated and/or nonhydrogenated petrolatum, paraffin and/or stearin. In embodiment variant, the hydrophobic substance is hydrogenated petrolatum.

In embodiments, the hydrophobic substance is chosen from the group consisting of hydrogenated and/or nonhydrogenated oils and/or fats.

In embodiments, the hydrophobic substance is chosen from the group consisting of natural and/or synthetic waxes, for example carnauba wax, jojoba oil, hard paraffin and so-called microcrystalline wax (waxes extracted from petroleum), and silicone waxes (waxes obtained by synthesis). In embodiments, the hydrophobic substance is beeswax. Beeswax is principally composed of an ester of ethylene glycol and two fatty acids. In other embodiments, the hydrophobic substance is carnauba wax.

The disclosure is also directed to a tampon configured so that the part in which the expansion and/or the absorption are limited is situated at the proximal end of the tampon.

In embodiments, the part in which the expansion and/or the absorption are limited is situated at a distance of more than 0.5 mm from the proximal end of the tampon.

In embodiments, the zone in which the expansion and/or the absorption is limited forms a spiral inside and outside the tampon.

In embodiments, the tampon additionally comprises another zone in which the expansion and/or the absorption are limited, situated half way between the two ends of the tampon.

In embodiments, the tampon additionally comprises another part in which the expansion and/or the absorption are limited, forming a spiral outside the tampon. In such embodiments, and in contrast to the tampon described and shown in EP 1 383 453, the spiral shape obtained may be nondeformable or only minimally deformable, even after saturation of the tampon with a hydrophilic substance, and this spiral shape makes it possible to channel the flow and thus avoid flow of fluid along the vaginal walls, and also to increase the time of contact between the absorbent material and the flow, thus permitting improved absorption and a greater capacity of the tampon.

The tampons may be impregnated with the solid hydrophobic substance whose melting point is greater than 37°C in the melted state and before and/or after formation of the tampon. When the substance is applied before formation of the tampon, it is applied to the band before folding and/or compression and/or rolling of the band.

A zone formed by a line measuring several tenths of a millimeter to several millimeters is applied continuously or discontinuously to the front face and/or rear face of the band, optionally in several zones, and optionally nonsymmetrically. The zones of impregnation can be arranged on what will become the lower part, middle part, and/or the upper part of the tampon in order to create one or more reservoirs with these successive barriers.

The zones can be arranged in such a way that, after folding and/or compression of the band, a spiral shape is obtained that is nondeformable or only minimally deformable, even after saturation of the tampon with a hydrophilic substance. This spiral shape makes it possible to channel the flow, and thus avoid flow of fluid along the vaginal walls, and also to increase the time of contact between the absorbent material and the flow, thus improving absorption and capacity of the tampon.

In embodiments, the tampon comprises an impregnation zone in the form of an upturned funnel which, by diverting the flow from the center of the tampon, makes it possible to avoid accumulation of fluid at the site of fixation of a withdrawal thread and to avoid leaks due to the presence of this withdrawal thread.

In embodiments, the means for limiting the expansion and/or the absorption are formed by at least one restriction member enclosing the corresponding zone of the body of the tampon, while allowing the zone to be maintained in its initial shape when the body of the tampon inflates under the effect of the absorption.

In embodiments, the restriction member can be made in the form of a ring, a washer, a string, a collar, or one or more foldable bands made of permeable material.

In embodiments, the restriction member comprises, on its outer face, at least one smooth zone that makes it possible to limit the friction during removal of the tampon, so as to avoid any injury.

In embodiments, the restriction member has a spiral shape either on the distal part of the tampon or on the proximal part of the tampon.

Advantageously, the ring is formed with the protector of the tampon.

In embodiments, each restrictor band comprises at least one orifice near each of its two ends, the orifice having a diameter that is substantially equal to that of the compressed tampon. Where appropriate, the restrictor band may comprise at least a third orifice in the central part for passage of the withdrawal thread.

In embodiments, the tampon comprises at least two superposed restrictor bands arranged perpendicular to each other before folding.

In embodiments, the restrictor band is designed to have a length that ensures the restrictor band does not disturb the natural development of the geometry of the tampon body when the tampon is placed in contact with a fluid that is to be absorbed.

The disclosure also relates to a strip of absorbent material comprising zones in which the absorbent material is partially or totally impregnated with a solid hydrophobic substance whose melting point is greater than 37°C.

The disclosure is also directed to a method for producing a tampon, wherein the method finally comprises, after a step of folding and/or of compression and/or of impregnation of an absorbent material such as an absorbent strip including a hydrophobic substance, a step of heating to a temperature above the melting point of the hydrophobic substance, making it possible to bind the impregnated zones.

The disclosure is also directed to a tampon comprising a saturation indicator at its proximale end. In embodiments, the indicator is formed by the proximal zone situated at the proximal end of the tampon comprising at least one zone in which the expansion and/or the absorption are limited, the zone being formed by partial or total impregnation of the absorbent material of the tampon with a solid hydrophobic substance whose melting point is greater than 37°C. In embodiments, the indicator is formed by the proximal zone situated at the proximal end of the tampon comprising at least one zone in which the expansion and/or the absorption are limited, the zone being formed by placement of a restriction member, for example in the form of a ring, string, collar or restrictor band(s) encircling the tampon and allowing it to be maintained in its initial shape in the zone in question, when the tampon comes to inflate under the effect of the absorption. The various embodiments may be separate or in combination with one or more of the other embodiments.

### BRIEF DESCRIPTION OF THE DRAWINGS

Embodiments will be better understood from the detailed description given below and by reference to the attached drawings, in which:
Figures 1 and 2 are diagrammatic views of two strips of absorbent material;
Figures 3 and 3a are diagrammatic views of two other strips of absorbent material during the injection of the hydrophobic substance;
Figure 4 is a diagrammatic view of another strip of absorbent material when laid out flat;
Figures 5 and 5a are diagrammatic views, in longitudinal section, of a tampon according to the embodiment of Figure 16 before and after heating of the hydrophobic substance;
Figure 6 is a diagrammatic view of the machine for producing a tampon according to the embodiment of Figure 16;
Figures 7 and 8 are diagrammatic views of a first strip of absorbent material and of a second strip of absorbent material, respectively, when laid out flat;
Figures 7a and 8a are perspective views of the tampons obtained respectively from the strips of absorbent material shown in Figures 7 and 8;
Figure 9 is a diagrammatic view of a third strip of absorbent material;
Figures 10, 11 and 12 are diagrammatic views of a fourth strip of absorbent material, a fifth strip of absorbent material and a sixth strip of absorbent material, respectively, when laid out flat;
Figures 10a, 11 a and 12a are perspective views of the tampons obtained respectively from the strip of absorbent material shown in Figures 10, 11 and 12;
Figure 13 is a diagrammatic view of another strip of absorbent material when laid out flat;
Figure 13a is a perspective view of the tampon obtained from the strip of absorbent material shown in Figure 13;
Figure 14 is a diagrammatic view of four other illustrative embodiments of a strip of absorbent material;
Figures 15 and 15a are diagrammatic perspective views of two tampons according to the embodiment of Figure 16 during the phase of injection of the hydrophobic substance;
Figures 16 and 16a are diagrammatic perspective views of a tampon according to an embodiment, before use (Figure 16) and after use (Figure 16a);
Figure 17 is a diagrammatic view of a traditional tampon and Figure 17a is a diagrammatic view of a tampon according to the embodiment of Figure 16 during the withdrawal phase;
Figure 18 is a diagrammatic view of a tampon according to another embodiment;
Figure 18a is a diagrammatic view of a tampon according to another embodiment;
Figures 19 and 19a are partial diagrammatic views, in longitudinal section, of two separate rings with which a tampon according to the embodiment of Figure 18 is equipped;
Figure 20 is a diagrammatic end view of a restriction member in the form of one or more washers;
Figures 20a and 20b are diagrammatic perspective views of another tampon equipped with restriction members shown in Figure 20, before and after use;
Figure 21 is a diagrammatic end view of a restriction member in the form of another type of washer;

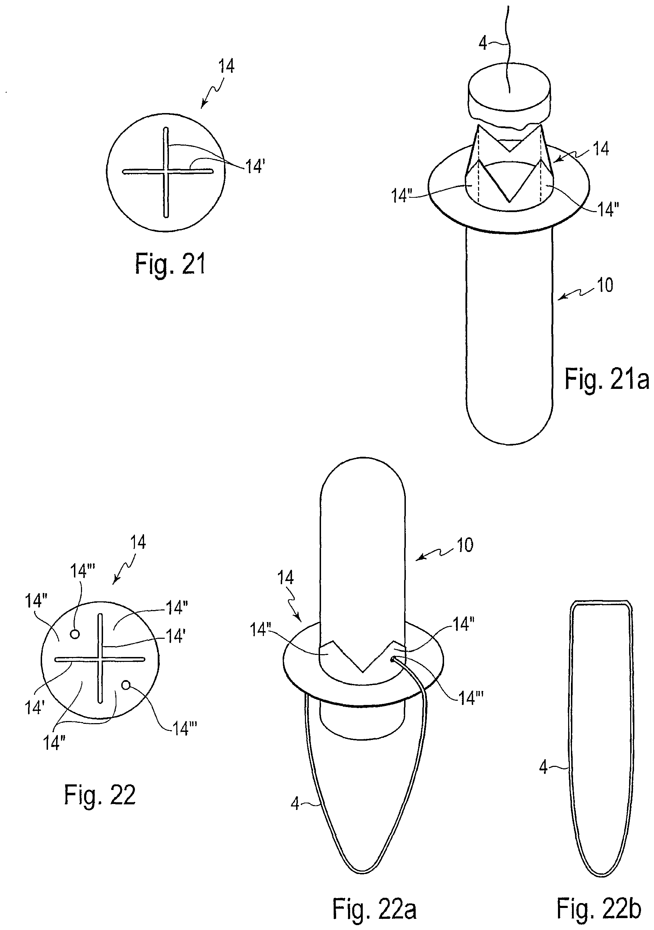

Figure 21 a is a diagrammatic perspective view of another tampon equipped with a restriction member shown in Figure 21;
Figure 22 is a diagrammatic end view of a restriction member in the form of another type of washer;
Figure 22a is a diagrammatic perspective view of another tampon equipped with a restriction member shown in Figure 22;
Figure 22b is a diagrammatic perspective view of the withdrawal thread with which the tampon shown in Figure 22a is equipped;

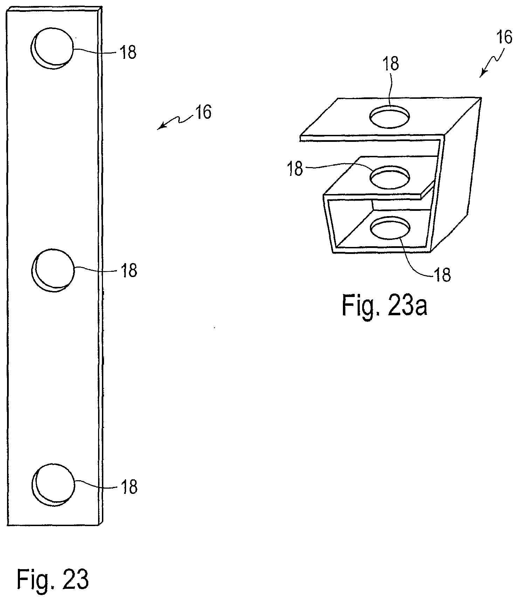

Figures 23 and 23 a are diagrammatic views of a restrictor band forming a restriction member, before folding (Figure 23) and after folding (Figure 23a);

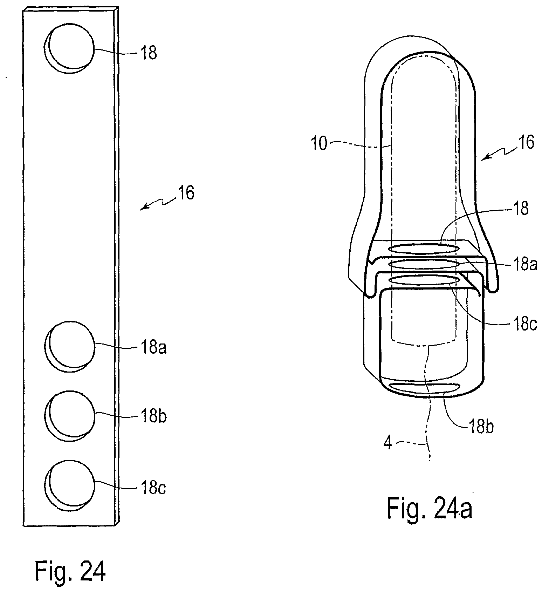

Figure 24 is a diagrammatic flat view of another restrictor band forming a restriction member;
Figure 24a is a diagrammatic perspective view of another tampon equipped with the restrictor band shown in Figure 24;

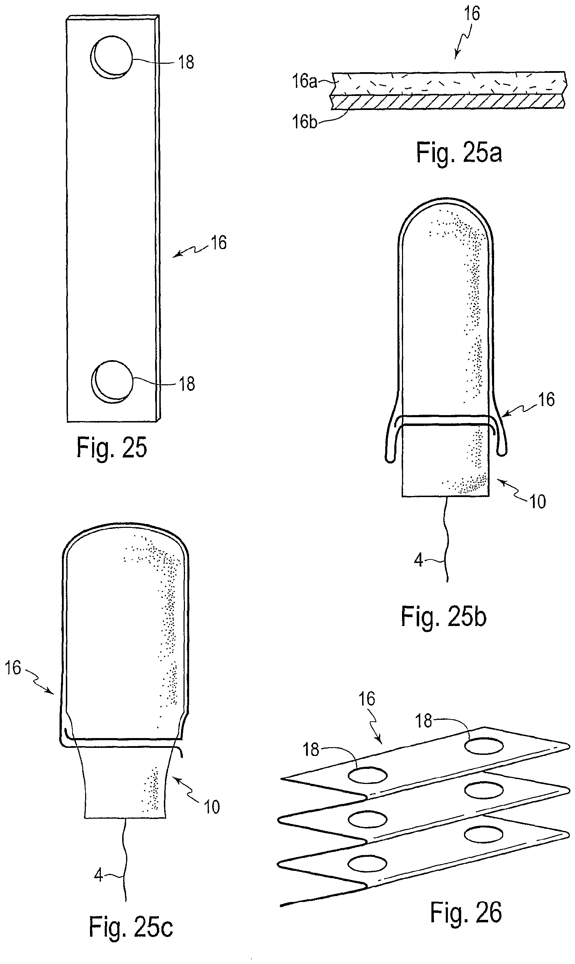

Figure 25 is a diagrammatic flat view of a restrictor band forming a restriction member;
Figure 25a is a diagrammatic view, in longitudinal section, of another restrictor band forming a restriction member;
Figures 25b and 25c are diagrammatic perspective views of another tampon equipped with the restrictor band shown in Figure 25, before and after use;
Figure 26 is a diagrammatic perspective view of another restrictor band forming a restriction member, after folding;

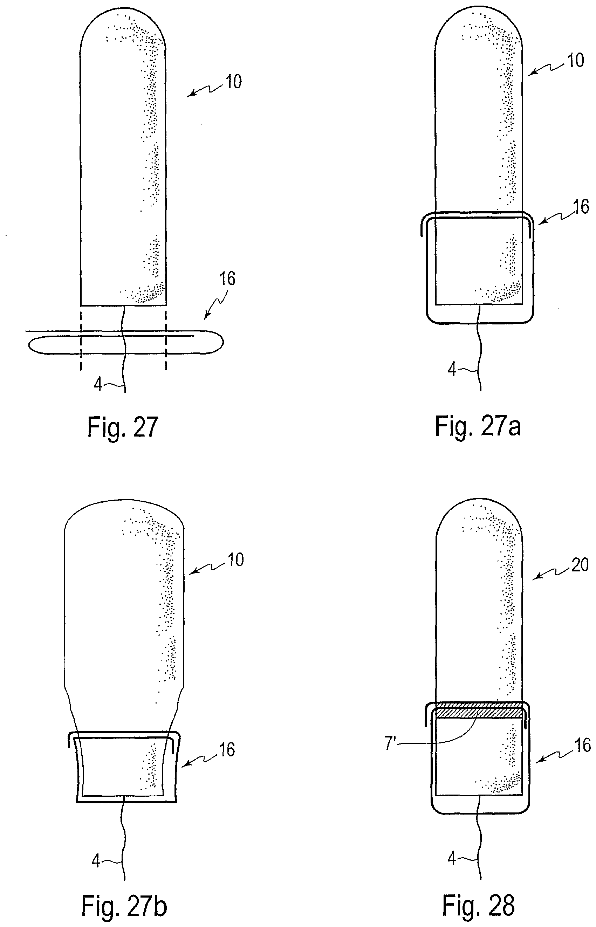

Figure 27 is a diagrammatic view of the placement of the restriction member, shown in Figure 23a, around a tampon;
Figure 27a is a diagrammatic perspective view of the tampon shown in Figure 27, once the restriction member is in place;
Figure 27b is a view similar to Figure 27a, after use;
Figure 28 is a diagrammatic perspective view of a tampon as shown in Figure 27a, additionally comprising a fluidic barrier formed with the aid of a hydrophobic substance;

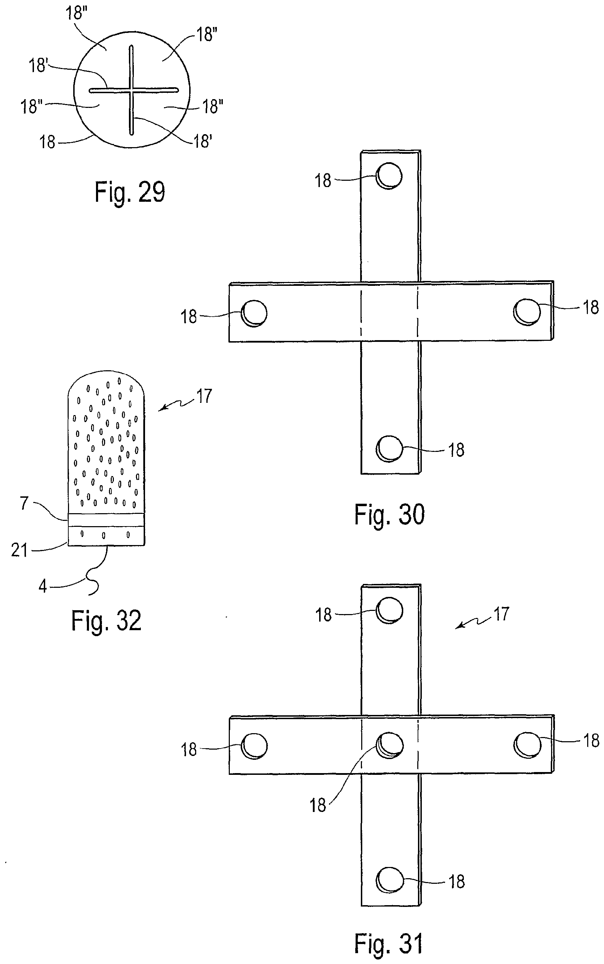

Figure 29 is a diagrammatic end view of an orifice according to another embodiment;
Figures 30 and 31 are diagrammatic views of two other restriction members that are cross-shaped; and
Figure 32 is a diagrammatic perspective view of a tampon with a saturation indicator.

### DETAILED DESCRIPTION OF EMBODIMENTS

In the following detailed description of the figures defined above, the same elements, or the elements performing identical functions, will keep the same reference numbers, so as to make various embodiments easier to understand. For example, reference number 1 is given to any tampon according to one embodiment, and reference number 10 is given to any tampon according to a second embodiment of the invention.

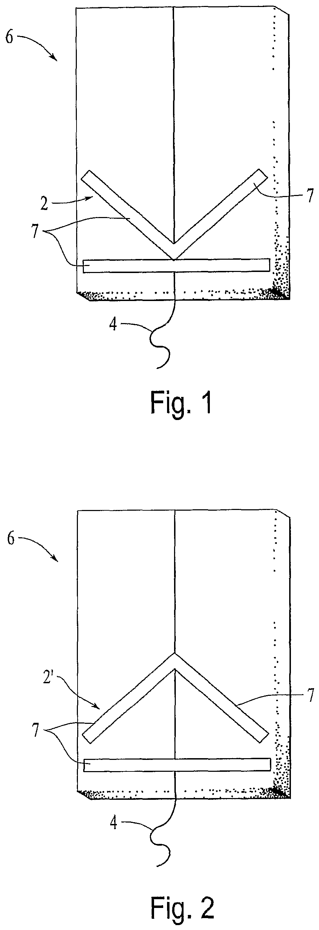

Tampons 1 are obtained from strip of absorbent material 6, such as are shown in Figures 1 and 2.

The form of the impregnation zone is specially designed as a function of the desired result.

In Figure 1, the impregnation zone forms a funnel 2 which channels the flow toward the proximal end of the tampon 1 and, for example, avoids the peripheral leaks due to the flow passing to the zone of contact between the walls (not shown) and the tampon 1.

In Figure 2, the impregnation zone is an upturned funnel 2' which, by diverting the flow from the center of the tampon 1, avoids accumulation of fluid at the site of fixation of the withdrawal thread 4 and avoids leaks due to the presence of the withdrawal thread 4.

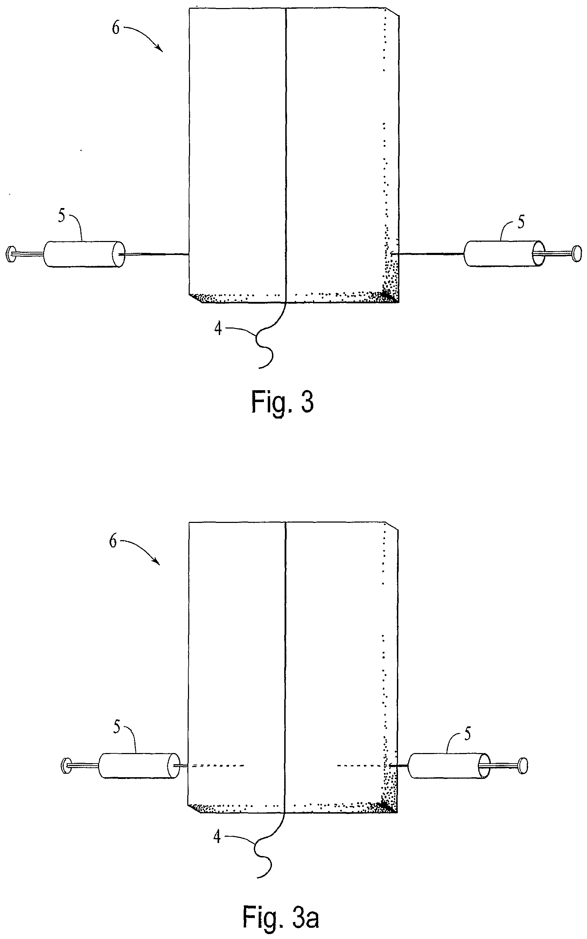

Figures 3 and 3 a show examples of injectors 5 for injecting a hydrophobic substance 7 into the thickness of a strip of absorbent material 6, the injection being carried out from the side.

After formation of the tampon 1, the zones of impregnation may be heated to temperatures above the melting point of the hydrophobic substance 7, so as to cause the hydrophobic substance 7 to melt and to better connect or affix the various layers that were saturated.

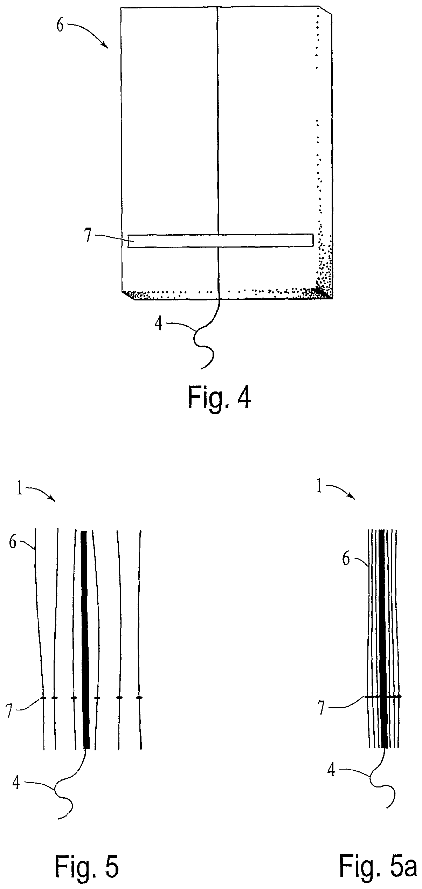

Figure 4 shows a strip of absorbent material 6 that will form a tampon 1 by means of folding or compression. The hydrophobic substance 7 is applied in the transverse direction on one face, or on both faces and, if appropriate, within the thickness of the strip of absorbent material 6.

Figures 5 and 5a show a schematic view of a folded tampon 1, with the points of application of the hydrophobic substance 7. Depending on the stage of formation of the tampon 1, the points of hydrophobic substance 7 may be joined to one another, for example by thermofusion at the time of folding, rolling and/or packaging, or may remain free.

The spacing, and hence the barrier and/or filter effect, is quantified by the intensity of the heating and also by the thickness of the impregnation and, consequently, by the quantity of hydrophobic substance 7 applied.

Thus, the parts that are not in direct contact with this strong heat source will be joined to a lesser extent to one another. The effect of this is to create passages that will slow the downward movement of the blood and will thus make it possible to increase the absorption time and capacity of the tampon 1 by an effect of filtration and deceleration of the flow, thereby making it possible to increase the time of contact between the flow and the absorbent material from which the tampon is made.

Moreover, it must be clearly understood that the dilation and/or expansion of the proximal part is able to be controlled in terms of time. The dilation and/or expansion is able to be produced with a delay effect, or may even be rendered impossible, depending on the quantity of the hydrophobic substance, the temperature of the latter, and the temperature during the step of thermofusion used in its production.

Methods for the production of a tampon 1 may further comprise, after a step of folding, and/or of compression and/or of impregnation, a heating step that makes it possible to bind the impregnated zones.

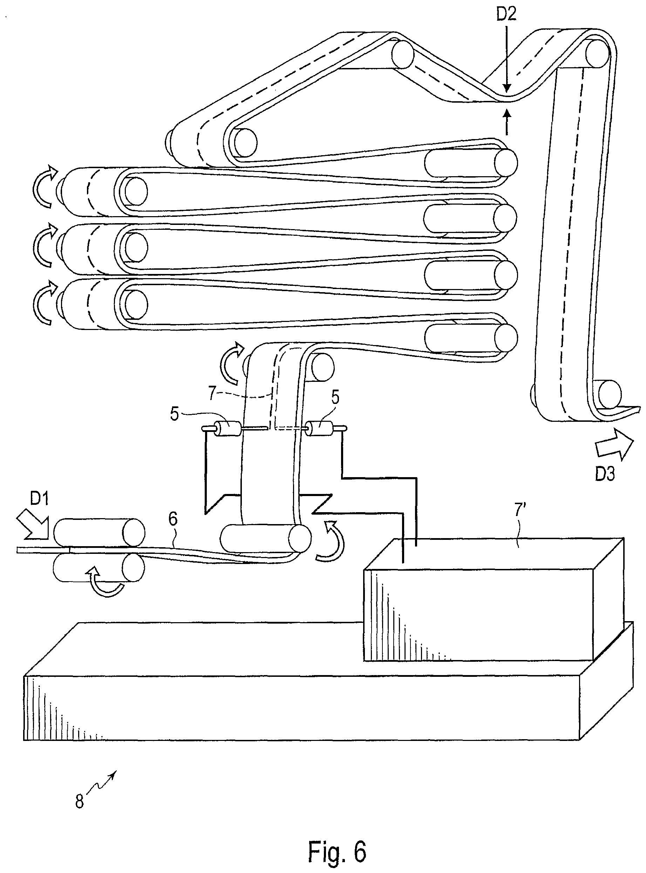

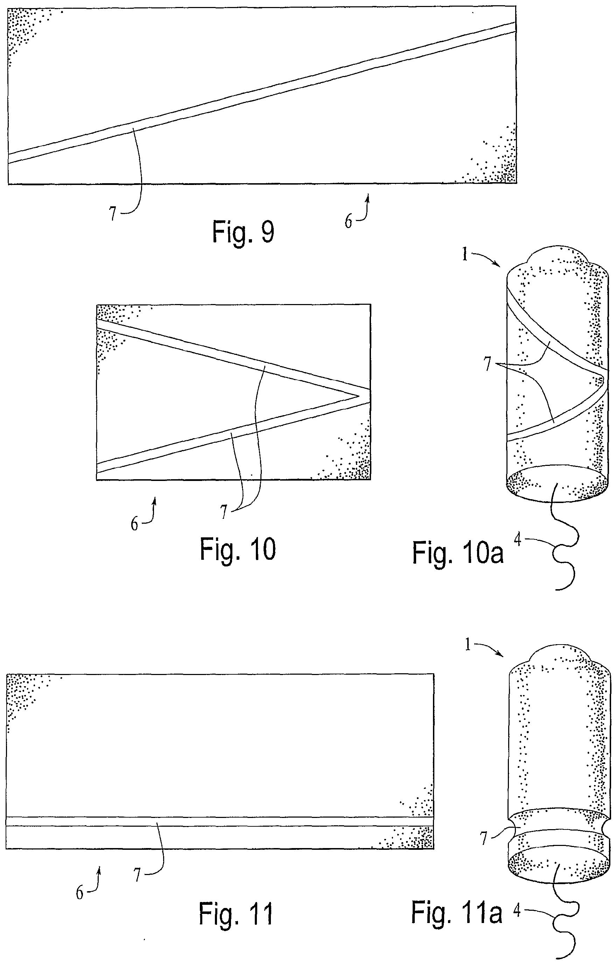

A tampon 1 may be produced using a machine 8 such as that shown schematically in Figure 6. More particularly, this machine 8 serves as a support for injecting the strip of absorbent material 6 with hydrophobic substance 7 on one of the two faces, or both of them, or within the thickness of the strip of absorbent material, with the aid of the injectors 5 that are connected to an injection device 7' for the hydrophobic substance 7. These injectors 5 are either single or multi-directional. For injections into the thickness of the strip of absorbent material 6, the injectors 5 are equipped with needles that penetrate the strip of absorbent material 6, as illustrated in Figures 3 and 3 a. The injectors 5 may be of the traditional pump type, which may be mounted on an "XY" table to configure the forms. The injectors 5 are optionally mobile, in order to apply the hydrophobic substance 7 by creating forms such as those illustrated in Figures 1, 2, 7-10, and 13.

The path traveled by the strip of absorbent material 6 from its arrival at D1 to the outlet D3 makes it possible to lengthen the time between the application of the hydrophobic substance 7 and the zone of assembly of the tampon 1, and thus permits stiffening of the hydrophobic substance 7. In embodiments, a jet of air may be applied to accelerate this stiffening. One observes at D2 a flexion in the strip of absorbent material 6 to control the traction of the strip of absorbent material 6, and at D3 the exit of the strip of absorbent material 6 for entry into the following assembly machine.

The hydrophobic substance 7 may also be placed in a very precise manner such that it is located only inside the tampon 1 at the time the latter is rolled up or folded, and not on the visible outer part of the tampon 1. The barrier is located inside the tampon 1, and the first layer will permit absorption of the lateral leaks flowing along the wall.

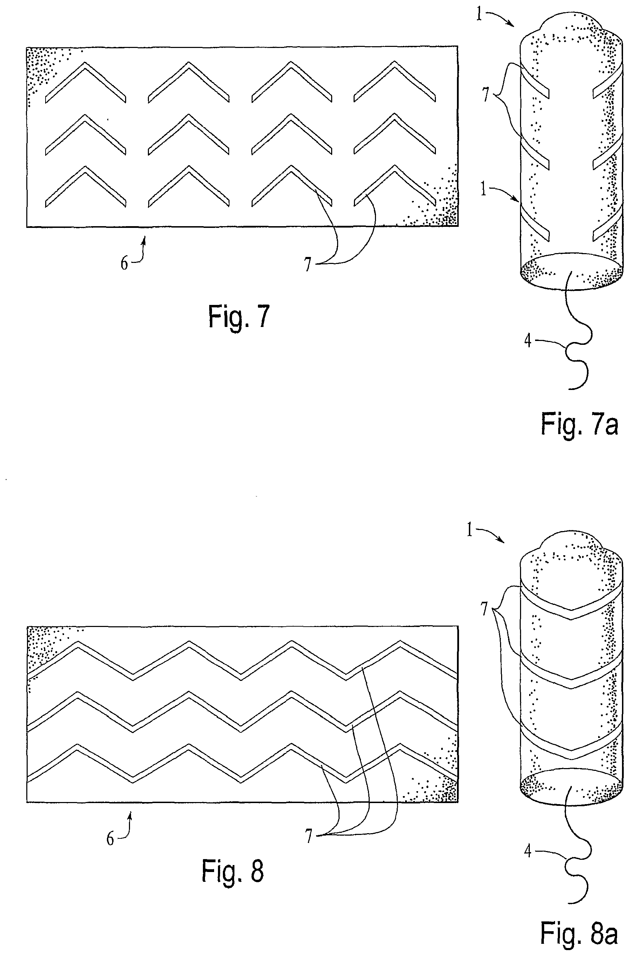

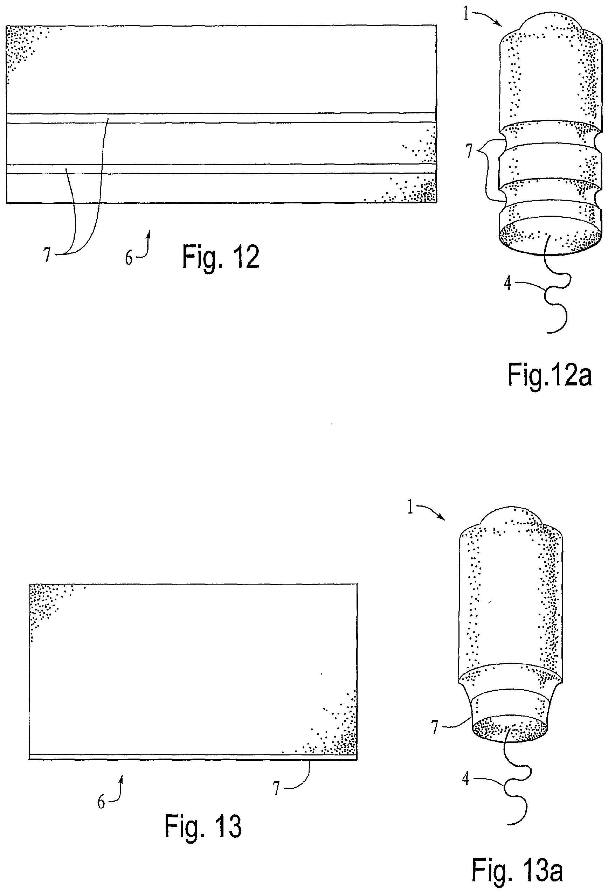

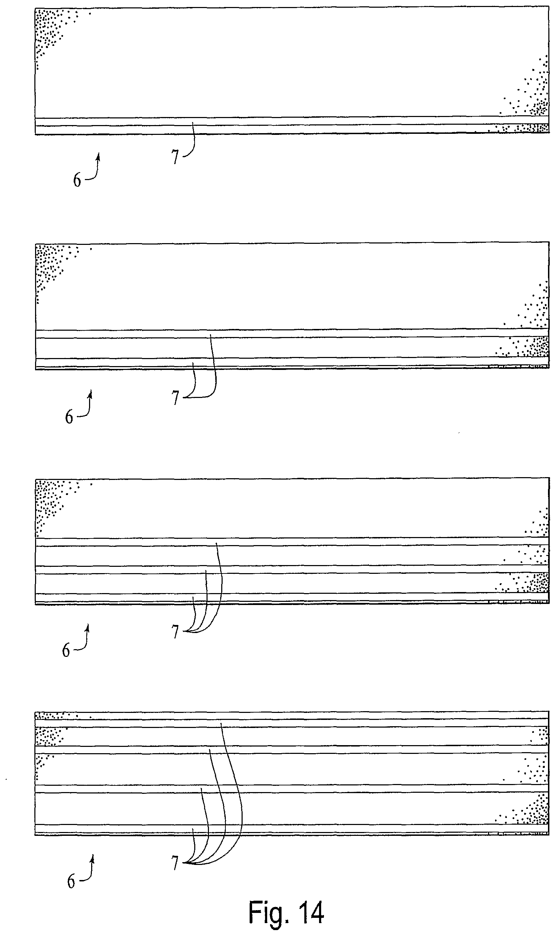

Different application variants are illustrated in Figures 7 to 12. Figures 13 to 15a schematically illustrate the results obtained and the methods of impregnation.

The application may be carried out on the tampon 1 when already formed, the melted hydrophobic substance 7 thus being applied with an injection or impregnation system, after protection of the zones that are not to be impregnated on the tampon 1 already formed.

It is also possible to soak the withdrawal thread 4 in the operating mode, either before being assembled or during the phase with the tampon 1.

A strip of absorbent material 6 of absorbent material may comprise zones in which the absorbent material is partially or totally impregnated with a solid hydrophobic substance 7 whose melting point is greater than 37°C. For example, the hydrophobic substances described herein may have a melting point equal to or greater than 38°C, 39°C, 40°C, 41 °C, 42°C, 43°C, 44°C, 45°C, or 46°C. In embodiments, the hydrophobic substance has a melting point equal to or greater than 47°C, 49°C, 51°C, 53°C, 55°C, 57°C, 59°C, 61 °C, or 63°C. In embodiments, the hydrophobic substance has a melting point equal to or greater than 68°C, 69°C, 70°C, 71 °C, 72°C, 73°C, 74°C, 75°C, or 76°C.

The hydrophobic substances preferably do not dissolve in bodily fluids and preferably have a melting point that is higher than the internal temperature of the human body (37°C) so that the hydrophobic substance remains solid while the tampon is in a vagina. Preferably, the hydrophobic substance has a melting point that is 45°C or higher so that the hydrophobic substance remains solid during shipping and/or storage of the tampons where the tampons may be exposed to temperatures greater or significantly greater than 37°C. However, it is preferred that the melting point of the hydrophobic substance does not exceed 120°C, and more preferably does not exceed 100°C, thus making it more economical to manufacture the tampons in terms of costs and time. The lower the melting temperature of the hydrophobic substance, the less energy and time required to melt the hydrophobic substance for impregnation of the absorbent material, and to allow or cause the impregnated hydrophobic substance to solidify.

In embodiments, the hydrophobic substance 7 impregnated on the strip of absorbent material 6 is chosen from the group consisting of hydrogenated and/or nonhydrogenated oils and/or fats.

In embodiments, the hydrophobic substance 7 impregnated on the strip of absorbent material 6 is chosen from the group consisting of natural and/or synthetic waxes.

In embodiments, the hydrophobic substance 7 impregnated on the strip of absorbent material 6 is hydrogenated petrolatum.

In embodiments, the hydrophobic substance 7 impregnated on the strip of absorbent material 6 is beeswax.

In embodiments, the hydrophobic substance 7 impregnated on the strip of absorbent material 6 is carnauba wax.

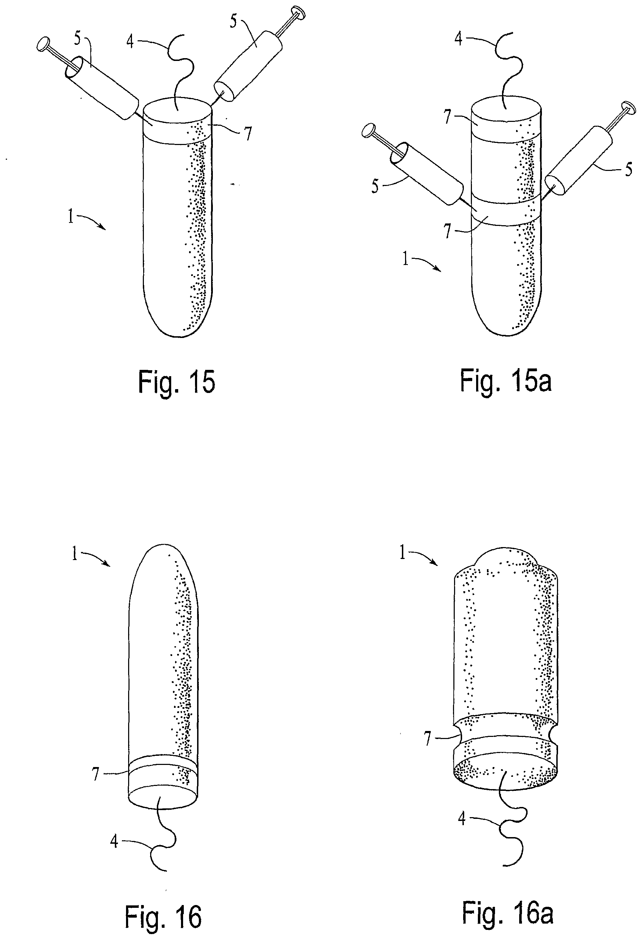

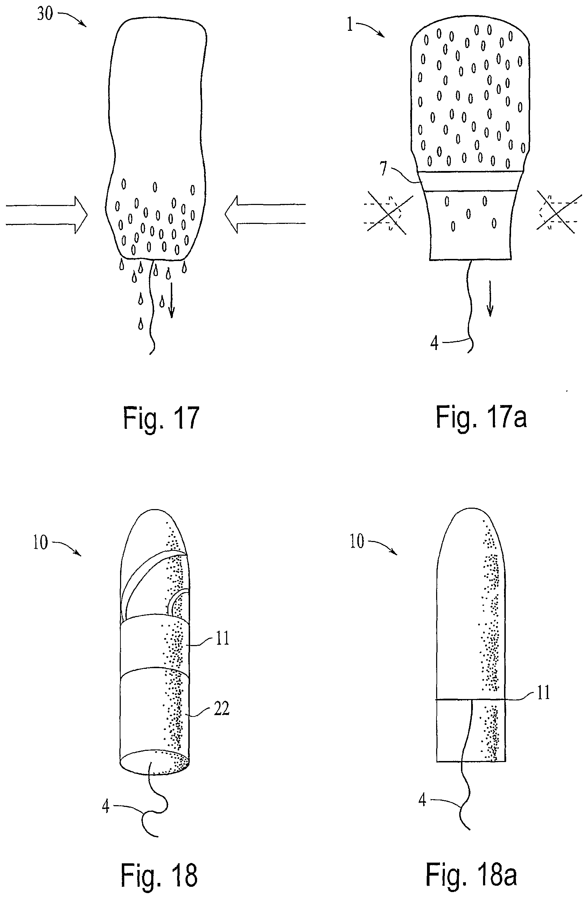

The mode of functioning of the absorption of the tampon 1 is illustrated in Figures 16, 16a and 17a. The absorption is improved by the barrier effect provided by the impregnation with the hydrophobic substance 7. This therefore makes it possible to reduce the first leaks. The zones that are impregnated are nondeformable or are minimally deformable, and they are not expanded, or are only minimally expanded, by the absorption of the flow of fluid. Moreover, such a tampon 1 may be designed to have an improved fluid absorption capacity of up to about 60%.

As is shown more specifically in Figure 17, a traditional tampon 30 will tend to allow an untimely quantity of blood to escape via the proximal end and the withdrawal thread, on account of pressure exerted on the tampon by the muscles near the vaginal opening during removal of the tampon 30. This pressure is represented schematically by the white arrows in Figure 17. By contrast, a tampon 1 configured with a fluidic barrier formed by a hydrophobic substance 7 as disclosed herein and as shown in Figure 17a, counteracts this phenomenon by reducing the amount of blood that flows into the proximal area of the tampon during withdrawal of the tampon.

When the impregnation is partial and/or the tampon 1 does not undergo heating after formation of the tampon 1 with preimpregnated strip of absorbent material 6, and/or after the impregnation, the barrier formed simply has a filtering effect and makes it possible to increase the absorption by increasing the contact time between the flow and the absorbent material from which the tampon 1 is made.

According to a second embodiment of a tampon 10, the means for limiting the expansion and/or the absorption are formed by at least one restriction member 11 encircling the corresponding zone of the body and allowing the zone to be maintained in its initial shape when the body comes to inflate under the effect of the absorption. This restriction member 11 may, for example, be in the form of a ring, a string or a collar encircling the body of the tampon 10, as is illustrated in Figure 18.

In an embodiment, the zone that is compressed or whose expansion and/or absorption are limited, functions as a means of reducing the passage of the fluid. The zone has a spiral shape either on the distal part of the tampon 10 or on the proximal part of the tampon 10.

In another embodiment, the restriction member 11 may be formed with a string, where at least a portion of the string is wrapped, tied, fastened, or otherwise affixed around the body of the tampon. As shown schematically in Figure 18a, a withdrawal thread 4 may also be connected to and/or function as the restriction member 11.

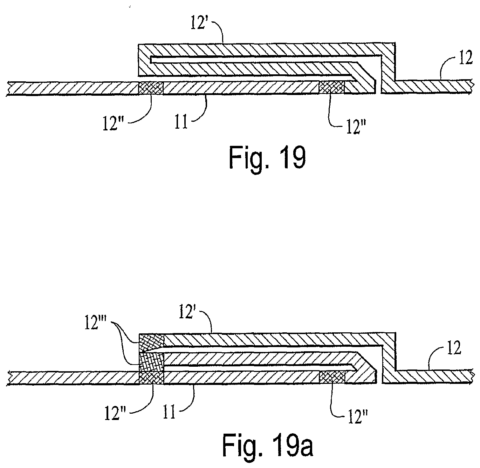

In another embodiment, the ring forming the restriction member 11 may be formed with the protector 12 or packaging of the tampon 10, such as a wrapper, which is cut or pre-slit or breakable so that the ring is separable from the protector 12 or packaging of the tampon 10, as is illustrated in Figures 19 and 19a.

This protector 12 has a tab 12' for guaranteeing the protection of the ring 11 intended to remain in place after the insertion. During use of the tampon 10, the user takes hold of this tab 12' in order to fragment the protector 12 at the area of two weld points or bonding points 12". The ring 11 will then be formed by the remaining part of the protector 12 around the tampon 10.

Moreover, and as is shown in Figure 19a, the tab 12' may be reattached to the protector 12 via weld points or bonding points 12"'. This variant is particularly advantageous from the point of view of hygiene, because the ring 11 is free of any prior contact with the exterior before detachment of the protector 12.

The position of the ring 11 may be centered with respect to the distal and/or proximal ends and/or offset toward one of the ends.

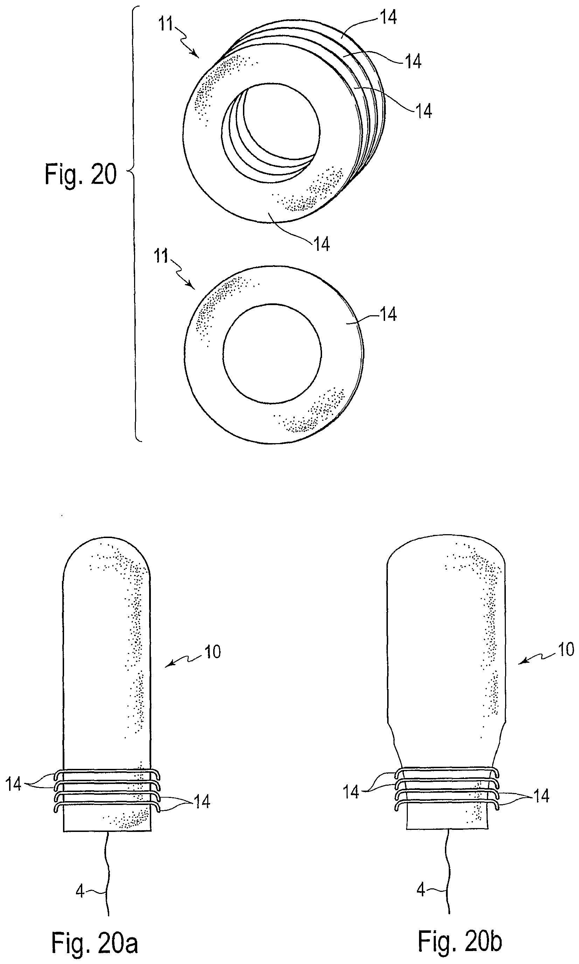

As is shown in Figures 20 and 20a, the ring may be formed by a single washer 14 or by a plurality of stacked washers 14, such as 2, 3, 4, 5, 6, 7, 8, 9, or 10 or more washers. The orifice of each washer 14 has a diameter substantially equal to that of the compressed body of the tampon 10. Figures 20a and 20b show a ring having four stacked washers 14.

As is shown in Figure 21, the orifice of each washer 14 is preferably formed by making two slits 14' perpendicular to each other, the effect of which is to delimit four substantially triangular flaps 14" which are oriented upward or downward depending on whether the washer 14 is introduced via the top or bottom of the tampon 10. However, the orifice may be formed by two slits that are not perpendicular to each other, or by more than two slits. In Figure 21a, the washer 14 has been slid on from the bottom of the tampon 10. The washers 14 may be secured to the tampon by affixing one or more of the triangular flaps 14" to the tampon by hot pressing, hot melting, sewing, or any other method, as schematically depicted by the dashed lines in Figure 21a.

Moreover, as is shown in Figure 22, two opposite flaps 14" may each comprise a hole 14"' in such a way as to allow the passage of the withdrawal thread 4 shown in Figure 22b. In Figure 22a, the washer 14 thus formed has been slid on from the top of the tampon 10.

Referring to Figures 23 to 31, it is noted that each restriction member may be made of a permeable material in the form of a restrictor band 16 or in the form of a cross 17 prior to folding.

More precisely, a restrictor band 16 for the upper part of the tampon 10 has at least one nondeformable or minimally deformable orifice 18 near each of its two ends, and the diameter of this orifice 18 is chosen such as to be equal to that of the body of the compressed tampon 10. Thus, once the tampon 10 is in place, the body of the tampon will inflate under the effect of the absorption, while the zone of the body of the tampon encircled by the edge of the orifices 18 will remain compressed. These orifices 18 may therefore actually play the role of a restriction member, prohibiting the tampon 10 from changing diameter in the desired zones.

As is shown more specifically in Figures 23 and 24, the restrictor band 16 has a supplementary central orifice 18 when it is a restrictor band intended for the lower part of the tampon 10 and requiring passage of the withdrawal thread 4.

It must also be clearly understood that the same restrictor band 16 may be used for the lower part and the upper part of the tampon 10, as is shown in Figures 24 and 24a. The restrictor band 16 in this case comprise four orifices 18, 18a, 18b, and 18c.

By contrast, the tampon 10 shown in Figure 25b comprises a restrictor band 16, illustrated in Figure 25, that has been folded and then inserted exclusively via the upper part of the tampon 10. Figure 25c shows the tampon 10 after use.

Another restrictor band 16 according to embodiments, as shown in Figure 25a, differs from the one described above in that it comprises, on the one hand, an inner absorbent layer 16a and, on the other hand, an outer impermeable layer 16b connected to the inner layer 16a.

As is shown in Figure 26, the restrictor band 16 may be formed by folding in an accordion configuration.

As is shown in Figure 27, the restrictor band 16 is folded in such a way as to align the orifices 18, with a view to then being positioned around the tampon 10, either via the top or via the bottom of the tampon 10, by inserting the body of the tampon 10 through the orifices 18. In Figure 27a, this restrictor band 16 is thus introduced via the bottom of the tampon 10.

Advantageously, and as is shown in particular in Figure 27a, the restrictor band 16 is preferably long enough to leave enough "slack" such that the tampon 10 can dilate. After use, and as is shown in Figure 27b, the restrictor band 16 will enclose the tampon 10 at the bottom part. In this embodiment, reduced or no friction occurs in the area of the vaginal wall during spasms. Indeed, the slight movement of translation of the tampon due to each spasm does not cause any movement of the restrictor band 16 itself because of the "slack," which allows the restrictor band 16 to remain motionless in relation to the vaginal wall. Furthermore, withdrawal of the tampon is easier because it happens schematically in two steps due to the "slack." In other words, the "slack" in the restrictor band 16 allows some freedom of movement of the body of the tampon with respect to the restrictor band 16. Thus, when withdrawing the device, the body of the tampon will move first until it has been moved a sufficient distance that the "slack" in the restrictor band 16 is taken up, whereby the movement of the body of the tampon exerts enough tension on the restrictor band 16 that the body of the tampon and the restrictor band 16 move relative to the walls of the vagina at an equal rate. Also, the pulling force on the body of the tampon elongates the tampon in the axial direction, which decreases the radial diameter of the body of the tampon. As a result, the body of the tampon exerts less radial force on the restrictor band 16, which reduces the frictional force between the walls of the vagina and the restrictor band 16. Accordingly, the longitudinal forces that must be applied to the tampon to withdraw the device are lower than those required with a traditional tampon.

For still greater efficacy, it must be clearly understood that the limitation of the expansion and/or of the absorption of one or more zones of a tampon 20 according to the invention is able to be obtained by simultaneous use, as shown in Figure 28, of a fluidic barrier 7', formed with the aid of a hydrophobic substance 7, and also of a restriction member, such as a restrictor band 16. The fluidic barrier 7' and the zone formed by a restriction member may be located in a proximate area of the tampon, and may be located at substantially the same distance from the proximal end of the tampon.

As is shown in Figure 29, each orifice 18 of the restrictor band 16 may be formed by making two slits 18' perpendicular to each other, the effect of which is to delimit four substantially triangular flaps 18" which is oriented upward or downward depending on whether the orifice 18 is introduced via the top or via the bottom of the tampon 10. However, the orifice may be formed by two slits that are not perpendicular to each other, or by more than two slits. A restrictor band 16 may be secured to the tampon by affixing one or more of the triangular flaps 18" to the tampon by hot pressing, hot melting, sewing, or any other method.

Of course, the material used to form the restrictor band 16 is chosen to be able to resist the pressure of the tampon 10, 20 in the area of the orifices 18.

In the case where the restriction member has the form of a cross 17, each of the four ends comprises an orifice 18, as is illustrated in Figure 30. If it is a restriction member for the lower part of the tampon 10, a supplementary central orifice 18 is formed for passage of the withdrawal thread 4, as shown in Figure 31.

It must also be clearly understood that this cross 17 may alternatively be produced with the aid of two separate restrictor bands 16 that are superposed in the area of their central parts and are arranged perpendicular to each other. In the case where this cross 17 is arranged in the lower part of the tampon, the central orifices 18 of the two restrictor bands 16 are superposed in such a way as to permit passage of the withdrawal thread 4.

The restriction member, whether in the form of a restrictor band 16 or a cross 17, is designed to provide some "slack," such that it is able to adapt to the geometry of the body once it has developed in shape under the effect of the inflation associated with the absorption of the fluid, as is illustrated in particular in Figures 24 and 24a. For this reason, the tampon 10 is able to deploy fully in the upper part without being impeded by the restrictor band 16 or cross 17 arranged around it.

Generally speaking, increasing the number of orifices 18 makes it possible to reduce the stresses that have to be withstood by each orifice 18.

In embodiments, the tampon comprises a saturation indicator at its proximal end. A saturation indicator is understood as a means allowing the user to verify the state of saturation or nonsaturation of the tampon by simple contact.

As is illustrated in Figure 32, the proximal zone 21 situated at the proximal end of the tampon 1 comprises at least one zone in which the expansion and/or the absorption are limited, this zone being formed by partial or total impregnation of the absorbent material of the tampon 1 with a solid hydrophobic substance 7 whose melting point is greater than 37°C. This proximal zone 21 may then serve as a saturation indicator zone.

In fact, because of the presence of the zone in which the expansion and/or the absorption are limited, the proximal zone 21 is soaked only when the tampon 1 is saturated. This proximal zone 21 may thus be used as a saturation indicator. If the proximal zone 21 is neither soaked nor inflated then this indicates to the user that the tampon 1 is not saturated.

In embodiments, the tampon 10 illustrated in Figure 17 is notable in that the saturation indicator is formed by the proximal zone 22 situated at the proximal end of the tampon 10 comprising at least one zone in which the expansion and/or the absorption are limited by placement of at least one restriction member 11, for example in the form of a ring, string, collar or one or more restrictor bands encircling the zone of the body of the tampon and allowing it to be maintained at this location in its initial shape when the body of the tampon inflates under the effect of the absorption.

Although the invention has been described in connection with particular illustrative embodiments, it will be clear that it is not in any way limited to these embodiments and that it covers all the technical equivalents of the means described, and their combinations, insofar as these come within the scope of the invention.

## Claims

1. Sanitary tampon, comprising
a body (1) of absorbent material (6) and a withdrawal thread (4) fixed to the absor-bent material (6),
wherein the body (1) has a distal end and an opposite proximal end and the withdrawal thread (4) extends from the proximal end of the body (1),
wherein the body (1) of the tampon is formed from a strip of the absorbent material (6) by means of folding or compression,
**characterized in that**,
the strip of absorbent material (6) that is formed into the body (1) of the tampon comprises at least one zone (7, 7') in which the absorbent material (6) is partially or totally impregnated with a solid hydrophobic substance having a melting point that is greater than 37°C, wherein the solid hydrophobic substance limits expansion and/or absorption by the zone when the body of the tampon contacts an absorbable fluid.

2. The tampon according to claim 1, **characterized in that** the hydrophobic substance is selected from the group consisting of hydrogenat-ed and nonhydrogenated oils and fats.

3. The tampon according to claim 1, **characterized in that** the hydrophobic substance is selected from the group consisting of natural and synthetic waxes.

4. The tampon according to claim 1, **characterized in that** the hydrophobic substance is hydrogenated petrolatum.

5. The tampon according to claim 1, **characterized in that** the hydrophobic substance is beeswax or carnauba wax.

6. The tampon according to any one of the preceding claims, **characterized in that**
the expansion and/or absorption are limited by placement of at least one restriction member (11).

7. The tampon according to claim 6, **characterized in that** the restriction member (11) comprises at least one member selected from the group consisting of a ring, a washer, a string, a collar, and a foldable restrictor band.

8. The tampon according to claim 7, **characterized in that** an outer surface of the restriction member (11) comprises at least one smooth zone configured to reduce friction between the tampon (1, 10) and a vagina during removal of the tampon (1, 10).

9. The tampon according to claim 7, **characterized in that** the restriction member (11) has a spiral shape.

10. The tampon according to claim 7, **characterized in that** the restriction member (11) comprises a ring that comprises a separable portion of a wrapper on the tampon (1, 10).

11. The tampon according to claim 7, **characterized in that** the restriction member (11) comprises at least one restrictor band having two ends, and said restrictor band comprises at least one orifice near each of the two ends of the restrictor band, the at least one said orifice having a diameter that is substantially equal to a diameter of the tampon (1, 10) when the tampon (1, 10) is compressed.

## Patentansprüche

1. Hygienetampon, umfassend
einen Körper (1) aus saugfähigem Material (6) und einen Rückholfaden (4), der an dem saugfähigen Material (6) befestigt ist,
wobei der Körper (1) ein distales Ende und ein gegenüberliegendes proximales Ende aufweist und sich der Rückholfaden (4) vom proximalen Ende des Körpers (1) erstreckt,
wobei der Körper (1) des Tampons durch Falten oder Zusammenpressen aus einem Streifen aus saugfähigem Material (6) gebildet wird,
**dadurch gekennzeichnet, dass** der Streifen aus saugfähigem Material (6), der zum Körper (1) des Tampons geformt wird, mindestens eine Zone (7, 7') umfasst, in der das saugfähige Material (6) teilweise oder vollständig mit einer festen hydrophoben Substanz mit einem Schmelzpunkt über 37°C imprägniert ist, wobei die feste hydrophobe Substanz eine Expansion und/oder Absorption durch die Zone begrenzt, wenn der Körper des Tampons mit einer absarbierbaren Flüssigkeit in Berührung kommt.

2. Tampon nach Anspruch 1, **dadurch gekennzeichnet, dass**
die hydrophobe Substanz aus der aus hydrierten Ölen und Fetten bestehenden Gruppe ausgewählt ist.

3. Tampon nach Anspruch 1, **dadurch gekennzeichnet, dass**
die hydrophobe Substanz aus der aus natürlichen und synthetischen Wachsen bestehenden Gruppe ausgewählt ist.

4. Tampon nach Anspruch 1, **dadurch gekennzeichnet, dass**
die hydrophobe Substanz hydrierte Vaseline ist.

5. Tampon nach Anspruch 1, **dadurch gekennzeichnet, dass**
die hydrophobe Substanz Bienenwachs oder Carnaubawachs ist.

6. Tampon nach einem der vorhergehenden Ansprüche, **dadurch gekennzeichnet, dass**
die Expansion und/oder Absorption durch Platzierung mindestens eines Begrenzungselements (11) begrenzt werden.

7. Tampon nach Anspruch 6, **dadurch gekennzeichnet, dass**
das Begrenzungselement (11) mindestens ein Element umfasst, das aus der aus einem Ring, einer Unterlegscheibe, einer Schnur, einem Kragen und einem faltbaren Begrenzungsband bestehenden Gruppe ausgewählt ist.

8. Tampon nach Anspruch 7, **dadurch gekennzeichnet, dass**
eine Außenseite des Begrenzungselements (11) mindestens eine glatte Zone umfasst, die zur Reduzierung von Reibung zwischen dem Tampon (1, 10) und einer Vagina während der Entfernung des Tampons (1, 10) ausgelegt ist.

9. Tampon nach Anspruch 7, **dadurch gekennzeichnet, dass**
der Tampon (11) eine spiralförmige Gestalt aufweist.

10. Tampon nach Anspruch 7, **dadurch gekennzeichnet, dass**
das Begrenzungselement (11) einen Ring umfasst, der einen trennbaren Abschnitt einer Umhüllung auf dem Tampon (1, 10) umfasst.

11. Tampon nach Anspruch 7, **dadurch gekennzeichnet, dass**
das Begrenzungselement (11) mindestens ein Begrenzungsband mit zwei Enden umfasst und das Begrenzungsband mindestens eine Öffnung neben jedem der zwei Enden des Begrenzungsbands umfasst, wobei die mindestens eine Öffnung einen Durchmesser aufweist, der im Wesentlichen einem Durchmesser des Tampons (1, 10) gleicht, wenn der Tampon (1, 10) zusammengepresst ist.

## Revendications

1. Tampon périodique, comprenant :
un corps (1) de matériau absorbant (6) et un cordon de retrait (4) fixé au matériau absorbant (6),
dans lequel le corps (1) comporte une extrémité distale et une extrémité proximale opposée et le cordon de retrait (4) se prolonge à partir de l'extrémité proximale du corps (1),
dans lequel le corps (1) du tampon est formé à partir d'une bande du matériau absorbant (6) par pliage ou compression,
**caractérisé en ce que**
la bande de matériau absorbant (6) qui est formée pour produire le corps (1) du tampon comprend au moins une zone (7, 7') dans laquelle le matériau absorbant (6) est partiellement ou totalement imprégné d'une substance hydrophobe solide ayant un point de fusion qui est supérieur à 37 °C, la substance hydrophobe solide limitant l'expansion et/ou l'absorption par la zone quand le corps du tampon entre en contact avec un fluide absorbable.

2. Tampon selon la revendication 1, **caractérisé en ce que**
la substance hydrophobe est sélectionnée dans le groupe constitué d'huiles et de graisses hydrogénées et d'huiles et de graisses non hydrogénées.

3. Tampon selon la revendication 1, **caractérisé en ce que**
la substance hydrophobe est sélectionnée dans le groupe constitué de cires naturelles et de cires synthétiques.

4. Tampon selon la revendication 1, **caractérisé en ce que**
la substance hydrophobe est une vaseline hydrogénée.

5. Tampon selon la revendication 1, **caractérisé en ce que**
la substance hydrophobe est une cire d'abeille ou une cire de carnauba.

6. Tampon selon l'une quelconque des revendications précédentes, **caractérisé en ce que**
l'expansion et/ou l'absorption sont limitées par la mise en place d'au moins un élément de restriction (11).

7. Tampon selon la revendication 6, **caractérisé en ce que**
l'élément de restriction (11) comprend au moins un élément sélectionné dans le groupe constitué d'un anneau, d'une rondelle, d'un fil, d'un collier, et d'une bande de restriction repliable.

8. Tampon selon la revendication 7, **caractérisé en ce que**
une surface externe de l'élément de restriction (11) comprend au moins une zone lisse configurée pour réduire le frottement entre le tampon (1, 10) et un vagin pendant le retrait du tampon (1, 10).

9. Tampon selon la revendication 7, **caractérisé en ce que**
l'élément de restriction (11) est en forme de spirale.

10. Tampon selon la revendication 7, **caractérisé en ce que**
l'élément de restriction (11) comprend un anneau qui comprend une partie séparable d'un emballage sur le tampon (1, 10).

11. Tampon selon la revendication 7, **caractérisé en ce que**
l'élément de restriction (11) comprend au moins une bande de restriction comportant deux extrémités, et ladite bande de restriction comprend au moins un orifice près de chacune des deux extrémités de la bande de restriction, ledit au moins un orifice ayant un diamètre qui est sensiblement égal à un diamètre du tampon (1, 10) quand le tampon (1, 10) est comprimé.
